# FASCICULE DE BREVET EUROPEEN

(11) **EP 3 728 186 B1**
(45) Date de publication et mention de la délivrance du brevet: **17.11.2021**
(21) Numéro de dépôt: 18822354.9
(22) Date de dépôt: 20.12.2018
(51) Int. Cl.: C07C 319/02, C07C 321/04, B01J 23/28, B01J 23/00, B01J 37/02, B01J 37/18, B01J 37/20, B01J 37/08

(54) **PROCÉDÉ DE PRÉPARATION DE MÉTHYLMERCAPTAN**
VERFAHREN ZUR HERSTELLUNG VON METHYLMERCAPTAN
PROCESS FOR THE PREPARATION OF METHYL MERCAPTAN

(30) Priorité: 22.12.2017 FR 1763023
(43) Date de publication de la demande: 28.10.2020
(73) Titulaire: ARKEMA FRANCE, 92700 Colombes (FR); Université Lille 1 - Sciences Et Technologies, 59655 Villeneuve D'ascq Cedex (FR); CENTRE NATIONAL DE LA RECHERCHE SCIENTIFIQUE -CNRS-, 75016 Paris (FR)
(72) Inventeur: FRÉMY, Georges, 64390 Sauveterre de Béarn (FR); SALEMBIER, Hélori, 64000 Pau (FR); LAMONIER, Carole, 59280 Armentières (FR); BLANCHARD, Pascal, 62300 Lens (FR)
(74) Mandataire: Casalonga
(86) Numéro de dépôt international: PCT/EP2018/086086
(87) Numéro de publication internationale: WO 2019/122072

(56) Documents cités:
- WO-A2-2005/040082
- US-A1- 2010 094 059

## Description

La présente invention concerne un procédé de préparation de méthylmercaptan à partir d'oxyde de carbone, d'hydrogène sulfuré et d'hydrogène, ledit procédé mettant en œuvre un catalyseur spécifique à base de molybdène et de potassium.

Le méthylmercaptan présente un grand intérêt industriel, en particulier comme matière première de la synthèse de méthionine, acide aminé essentiel fortement utilisé dans l'alimentation animale. Le méthylmercaptan est également une matière première pour de nombreuses autres molécules, notamment le diméthyldisulfure (DMDS), additif de sulfuration de catalyseurs d'hydrotraitement de coupes pétrolières entre autres applications.

Le méthylmercaptan est couramment produit en gros tonnages industriellement à partir de méthanol et d'H₂S mais il peut se révéler intéressant économiquement de vouloir produire le méthylmercaptan directement à partir de monoxyde de carbone, d'hydrogène et d'hydrogène sulfuré selon la réaction suivante :

CO + 2H₂ + H₂S → CH₃SH + H₂O (1)

Le sous-produit principal de cette réaction est le dioxyde de carbone (CO₂). En effet, l'oxysulfure de carbone (COS) est considéré comme l'intermédiaire réactionnel qui mène au méthylmercaptan après hydrogénation selon les réactions suivantes :

CO + H₂S → COS + H₂ (2)

COS + 3H₂ → CH₃SH + H₂O (3)

Quant au CO₂, il est issu de plusieurs réactions parasites telles que :

CO + H₂O → CO₂ + H₂ (4)

COS + H₂O → CO₂ + H₂S (5)

2COS → CO₂ + CS₂ (6)

Ces réactions parasites qui consomment la matière première principale, le monoxyde de carbone, et l'intermédiaire réactionnel, l'oxysulfure de carbone, sont dues à la présence d'eau coproduite au cours de la synthèse de méthylmercaptan.

Comme décrit dans les demandes EP0171312 et US2008/262270, le dioxyde de carbone peut éventuellement être recyclé pour produire du méthylmercaptan selon la réaction suivante :

CO₂ + 3H₂ + H₂S → CH₃SH + 2H₂O (7).

Cette réaction est toutefois connue pour être plus lente que celle à partir du monoxyde de carbone. Il existe donc un intérêt à ce que la production de dioxyde de carbone soit la plus faible possible lors de la synthèse de méthylmercaptan.

Ainsi, les meilleures productivités en méthylmercaptan sont attendues par la voie gaz de synthèse selon la réaction (1), c'est-à-dire à partir de monoxyde de carbone, d'hydrogène et d'hydrogène sulfuré.

La demande de brevet US20070213564 décrit un procédé de production en continu de méthylmercaptan à partir de monoxyde de carbone, d'hydrogène et d'hydrogène sulfuré, ladite réaction étant catalysée par une famille de catalyseurs à base de K₂MoO₄ supportés sur silice. Selon ce procédé, 70% du monoxyde de carbone peut être converti avec des sélectivités en méthylmercaptan, dioxyde de carbone et oxysulfure de carbone égales respectivement à 49%, 43% et 8%.

La demande internationale WO2005/040082 décrit plusieurs catalyseurs et notamment un catalyseur comprenant un composant actif à base de Mo-O-K, un promoteur actif et éventuellement un support. Les catalyseurs exemplifiés sont de nature chimique différente tels que K₂MoO₄/Fe₂O3/NiO ou encore K₂MoO₄/CoO/CeO₂/SiO₂, chacun supporté sur de la silice. Le ratio de sélectivité CO₂/MeSH étant de 0,88.

La demande de brevet US20100094059 cite une famille de catalyseurs à base de TeO₂/K₂MoO₄ supportés sur un support poreux sélectionné parmi SiO₂, Al₂O₃, TiO₂, Al₂O₃-SiO₂, ZrO₂, zéolithes, matériaux carbonés, et un promoteur caractérisé exclusivement par l'oxyde de tellure (TeO₂). Il est démontré que la conversion en monoxyde de carbone, est de 59% pour une sélectivité en méthylmercaptan égale à 55%.

La demande de brevet US20100286448 divulgue une autre famille de catalyseurs composés d'un support poreux tel que SiO₂, TiO₂, les silico-alumines, les zéolithes et les nanotubes de carbone, sur lequel a été déposé électrolytiquement un métal. K₂MoO₄, ainsi qu'un autre oxyde métallique jouant le rôle de promoteur, sont ensuite imprégnés sur ce support. Ce catalyseur permet une conversion comprise entre 65% et 66% du monoxyde de carbone et une productivité en méthylmercaptan comprise entre 46% et 47%. Aucune donnée relative aux conditions opératoires et à la productivité du méthylmercaptan n'est précisée.

À partir de l'enseignement de ces documents, il a été observé que l'association de catalyseurs de structure spécifique, de promoteurs et de supports, chacun étant minutieusement sélectionné, permettait d'atteindre des rendements et des sélectivités améliorés par rapport aux procédés connus, tout en étant un procédé opéré de la manière la plus économique possible.

Malgré tous ces travaux de recherche, il reste un besoin pour un catalyseur facile à préparer et conduisant à de très bonnes sélectivités. Un des objectifs de la présente invention est de montrer qu'il est possible, grâce un catalyseur spécifique, et de formulation plus simple que celle connue de l'art antérieur, d'obtenir de meilleurs résultats de conversion en CO, une meilleure sélectivité et une meilleure productivité en méthylmercaptan, lors de la synthèse de celui-ci à partir de gaz de synthèse comprenant CO, H₂ et H₂S.

Ainsi, la présente invention concerne un perfectionnement à la production de méthylmercaptan (« CH₃SH » ou plus simplement « MeSH ») à partir de mélanges gazeux renfermant au moins un oxyde de carbone, de l'hydrogène et de l'hydrogène sulfuré.

Il a maintenant été découvert de manière surprenante que ce perfectionnement peut être obtenu grâce au catalyseur selon l'invention, qui est un catalyseur à base de molybdène et de potassium supporté sur zircone.

Le catalyseur selon l'invention est plus facile à préparer, notamment en raison du fait que le catalyseur ne comprend pas de promoteur. En outre, le catalyseur selon l'invention est également moins coûteux que ceux connus de l'art antérieur. Enfin, le catalyseur selon l'invention conduit à une meilleure conversion de l'oxyde de carbone, notamment du monoxyde de carbone, et une meilleure sélectivité en méthylmercaptan.
Selon un mode de réalisation préféré, le catalyseur selon l'invention est un catalyseur à base de molybdène et de potassium supportés sur zircone, qui ne comprend pas de promoteur. Selon un mode de réalisation, le catalyseur selon l'invention comprend des oxydes de potassium et de molybdène supportés sur zircone, et ne comprend pas de promoteur. Selon un autre mode de réalisation, le catalyseur selon l'invention est constitué d'oxydes de potassium et de molybdène supportés sur zircone.

Ainsi, selon un premier objet, la présente invention concerne un procédé de préparation de méthylmercaptan, ledit procédé comprenant au moins les étapes suivantes :
a) réaction d'un oxyde de carbone, d'hydrogène sulfuré (H₂S) et d'hydrogène (H₂) en présence d'un catalyseur à base de molybdène et de potassium supporté sur zircone ; ledit catalyseur ne comprenant pas de promoteur,
b) réaction d'hydrogénation de l'oxysulfure de carbone obtenu à l'étape a) en présence dudit hydrogène (H₂), pour former du méthylmercaptan (CH₃SH) et du sulfure d'hydrogène (H₂S),
c) éventuellement, recyclage dudit sulfure d'hydrogène (H₂S) formé à l'étape b) vers l'étape a), et
d) récupération du méthylmercaptan.

Ainsi, plus particulièrement, la présente invention concerne un procédé de préparation de méthylmercaptan, ledit procédé comprenant au moins les étapes suivantes :
a) réaction d'un oxyde de carbone, d'hydrogène sulfuré (H₂S) et d'hydrogène (H₂) en présence d'un catalyseur à base de molybdène et de potassium supportés sur zircone, ledit catalyseur ne comprenant pas de promoteur, pour former de l'oxysulfure de carbone,
b) réaction d'hydrogénation de l'oxysulfure de carbone obtenu à l'étape a) en présence dudit hydrogène (H₂), pour former du méthylmercaptan (CH₃SH) et du sulfure d'hydrogène (H₂S), ledit sulfure d'hydrogène étant issu de l'hydrolyse de l'oxysulfure de carbone, cette hydrolyse se faisant avec l'eau formée lors de l'hydrogénation de l'oxysulfure de carbone,
c) éventuellement, recyclage dudit sulfure d'hydrogène (H₂S) formé à l'étape b) vers l'étape a), et
d) récupération du méthylmercaptan.

Le procédé de préparation de méthylmercaptan selon la présente invention comprend donc une mise en contact des réactifs oxyde de carbone, hydrogène sulfuré (H₂S) et hydrogène (H₂) en présence d'un catalyseur à base de molybdène et de potassium supportés sur zircone, ledit catalyseur ne comprenant pas de promoteur, éventuellement un recyclage dudit sulfure d'hydrogène (H₂S) et la récupération du méthylmercaptan. La mise en contact desdits réactifs en présence dudit catalyseur permet de former l'oxysulfure de carbone.

Le procédé de la présente invention est réalisé à partir d'un oxyde de carbone, d'hydrogène et d'hydrogène sulfuré. L'oxyde de carbone est choisi parmi le monoxyde de carbone (CO) et le dioxyde de carbone (CO₂). De préférence l'oxyde de carbone est le monoxyde de carbone (CO), de sorte que le procédé de la présente invention est de préférence réalisé à partir d'un mélange d'oxyde de carbone, d'hydrogène et d'hydrogène sulfuré.

Selon un mode de réalisation particulièrement préféré, le catalyseur utilisé dans l'étape a) du procédé est le tétraoxomolybdate de potassium (K₂MoO₄) supporté sur zircone, et ne comprend pas de promoteur. Selon un mode de réalisation, le catalyseur utilisé dans l'étape a) du procédé est constitué de tétraoxomolybdate de potassium (K₂MoO₄) supporté sur zircone. L'utilisation de ce catalyseur permet en effet d'obtenir un taux élevé de conversion de l'oxyde de carbone, et en particulier du monoxyde de carbone, ainsi qu'un haut rendement et une haute sélectivité en méthylmercaptan.

Par ailleurs, le procédé selon l'invention est un procédé simple à mettre en œuvre, de faible éco-toxicité et économique.

Ainsi, le composant actif présent dans le catalyseur selon l'invention comprend du molybdène et du potassium au sein d'un même composant.

Le composant actif peut être obtenu par dépôt et calcination de précurseurs K₂MoO₄ ou (NH₄)₂MoO₄ additionnés de K₂CO₃ imprégné séparément sur le support. Il est également possible d'utiliser en tant que réactif l'heptamolybdate d'ammonium en présence d'un sel de potassium tel que par exemple le nitrate de potassium, le carbonate de potassium ou la potasse.

Ces composés sont des précurseurs des phases actives à base de molybdène, d'oxygène et de potassium (Mo-O-K), lesdites phases actives étant obtenues après prétraitement *in situ* des précurseurs, avec par exemple une procédure consistant dans une première étape en un séchage à l'azote, suivie par une sulfuration avec du sulfure d'hydrogène puis une étape de réduction/sulfuration avec un mélange H₂/H₂S.

Le support du catalyseur selon l'invention est de la zircone de formule ZrO₂. De préférence, le rapport pondéral du catalyseur sur zircone K₂MoO₄/ZrO₂ est compris entre 1% et 50%, de préférence de 1 à 30%, de préférence encore entre 5 et 35%, par exemple entre 5% et 25%.

L'activité catalytique du catalyseur utile pour le procédé de la présente invention peut encore être améliorée lorsque le support du catalyseur présente une aire spécifique supérieure à 30 m².g⁻¹. De préférence, le matériau support présente une aire spécifique d'au moins 50 m².g⁻¹.

La structure du support peut être une structure tridimensionnelle, de forme sphérique, cylindrique, en forme d'anneau, d'étoile, de granulats ou de toute autre forme tridimensionnelle, ou bien sous forme d'une poudre qui peut être pressée, extrudée, granulée, dans une forme tridimensionnelle.

Selon un mode de réalisation, le procédé selon l'invention est un procédé en deux étapes réactionnelles consécutives (étapes a) et b) ci-dessus), sans qu'il soit nécessaire d'effectuer une purification intermédiaire entre les deux étapes :
Étape a) :

   CO + H₂S → COS + H₂ (2)
Etape b):

   COS + 3H₂ → CH₃SH + H₂S (3')

L'étape b) correspond au bilan des deux réactions suivantes :

COS + 3H₂ → CH₃SH + H₂O (3)

COS + H₂O → CO₂ + H₂S (5)

En effet, la réaction d'hydrogénation de l'oxysulfure de carbone obtenu à l'étape a) en présence dudit hydrogène (H₂), forme du méthylmercaptan (CH₃SH) et du sulfure d'hydrogène (H₂S), ledit sulfure d'hydrogène étant issu de l'hydrolyse de l'oxysulfure de carbone donnant de l'H₂S et du CO₂, cette hydrolyse se faisant avec l'eau formée lors de l'hydrogénation de l'oxysulfure de carbone (et donnant donc du CH₃SH et de l'eau).

Selon un mode de réalisation, l'utilisation du catalyseur se fait dans les étapes a) et b) du procédé selon l'invention.

Le procédé selon l'invention est un procédé en deux étapes réactionnelles consécutives (étapes a) et b) ci-dessus), sans qu'il soit nécessaire d'effectuer une purification intermédiaire entre les deux étapes. D'une manière schématique, lorsque l'oxyde de carbone est le monoxyde de carbone, la première étape du procédé (étape a) est une réaction, effectuée de préférence à haute température, entre du monoxyde de carbone et du sulfure d'hydrogène (H₂S) selon la réaction (2) décrite précédemment :

CO + H₂S → COS + H₂ (2)

Dans la deuxième étape (étape b)), l'oxysulfure de carbone formé à l'étape a) est soumis à hydrogénation catalytique en partie avec l'hydrogène également formé à l'étape a), selon la réaction (3) décrite précédemment :

COS + 3H₂ → CH₃SH + H₂O (3)

Dans un mode de réalisation tout particulièrement avantageux de la présente invention, le sulfure d'hydrogène formé dans l'étape b) est recyclé dans l'étape a). Dans ce mode de réalisation, il est observé que la totalité du sulfure d'hydrogène formé peut ainsi être réutilisé dans l'étape a), ceci permettant d'éviter le stockage dudit sulfure d'hydrogène formé.

L'oxyde de carbone, le sulfure d'hydrogène et l'hydrogène sont avantageusement apportés en continu ou discontinu dans le ou les réacteurs dans lequel le procédé selon l'invention est mis en œuvre, en particulier selon que le procédé est mis en œuvre en continu ou en « batch ». Avantageusement, l'oxyde de carbone, l'hydrogène sulfuré et l'hydrogène sont sous forme liquide ou solide ou gazeuse, de préférence sous forme gazeuse.

En utilisant un catalyseur selon la présente invention, la température réactionnelle dans l'étape a) est avantageusement comprise entre 100 et 500°C, de préférence entre 200°C et 400°C, de préférence encore entre 250°C et 350°C.

La réaction de l'étape a) peut être indifféremment réalisée à pression atmosphérique, sous pression, voire sous dépression, l'homme du métier sachant adapter les conditions de pression réactionnelles selon la nature des réactifs mis en œuvre, les températures de réaction choisies, les vitesses de circulation des flux et les taux de conversion et les rendements visés.

De manière générale, l'étape a) peut être réalisée sous une pression comprise entre 50 mbars et 100 bars (soit entre 5.10³ et 1.10⁷ Pa), de préférence encore entre la pression atmosphérique et 50 bars (soit 5.10⁶ Pa), et avantageusement entre la pression atmosphérique et 15 bars (soit 15.10⁵ Pa).

De préférence, la réaction peut se dérouler dans des réacteurs tubulaires à lit fixe, multitubulaires, à micro-canaux, à paroi catalytique ou à lit fluidisé.

L'invention a également pour objet l'utilisation du catalyseur tel que défini ci-dessus pour la production de méthylmercaptan à partir d'oxyde de carbone, de sulfure d'hydrogène et d'hydrogène.

Les exemples suivants illustrent l'invention sans toutefois en limitée la portée définie par les revendications annexée à la description de la présente invention.

### Exemple 1 : Préparation du catalyseur K₂MoO₄ supporté sur zircone

Le catalyseur a été préparé par la méthode d'imprégnation à sec. Pour cela, une quantité de tétraoxomolybdate de potassium (K₂MoO₄) a été dissoute dans l'eau puis cette solution a été imprégnée sur la zircone. La teneur en Mo dans le catalyseur dépend de la solubilité de K₂MoO₄ et du volume poreux du support.

### Exemple 2 : Préparation du catalyseur K₂MoO₄ supporté sur silice

Le catalyseur a été préparé par la méthode d'imprégnation à sec. Pour cela, une quantité de tétraoxomolybdate de potassium (K₂MoO₄) a été dissoute dans l'eau puis cette solution a été imprégnée sur la silice. La teneur en Mo dans le catalyseur dépend de la solubilité de K₂MoO₄ et du volume poreux du support.

### Exemple 3 : Préparation du catalyseur K₂MoO₄ supporté sur dioxyde de titane.

Le catalyseur a été préparé par la méthode d'imprégnation à sec. Pour cela, une quantité de tétraoxomolybdate de potassium (K₂MoO₄) a été dissoute dans l'eau puis cette solution a été imprégnée sur le dioxyde de titane. La teneur en Mo dans le catalyseur dépend de la solubilité de K₂MoO₄ et du volume poreux du support.

### Exemple 4 : Préparation du catalyseur K₂MoO₄ supporté sur alumine.

Le catalyseur a été préparé par la méthode d'imprégnation à sec. Pour cela, une quantité de tétraoxomolybdate de potassium (K₂MoO₄) a été dissoute dans l'eau puis cette solution a été imprégnée sur l'alumine. La teneur en Mo dans le catalyseur dépend de la solubilité de K₂MoO₄ et du volume poreux du support.

### Exemple 5 : Test catalytique

Avant test, les catalyseurs ont été activés *in situ* avec une procédure consistant en une première étape de séchage avec balayage d'azote à 250°C, suivi par une sulfuration avec H₂S à la même température pendant 1 heure et finissant avec une étape de réduction/sulfuration avec H₂/H₂S à 350 °C pendant 1 heure.

La performance des catalyseurs est ensuite évaluée pour la réaction de production de méthylmercaptan dans un réacteur à lit fixe avec un volume de catalyseur de 3 mL, une température de 320°C, sous une pression de 10 bar (1 Mpa), avec une composition volumique de gaz d'alimentation CO/H₂/H₂S égale à 1/2/1 et un GHSV (« Gas Hourly Space Velocity ») égal à 1333 h⁻¹. Les réactifs et les produits sont analysés en ligne par chromatographie en phase gazeuse.

Les résultats obtenus pour ces 4 catalyseurs sont rassemblés dans le Tableau 1. Pour ces 4 essais, la teneur en molybdène sur le support est de 8% massique, soit 19,9% en K₂MoO₄.

**-- Tableau 1 --**

| ***Exemple*** | ***Catalyseur*** | ***Conversion CO (%)*** | ***Sélectivité molaire (%)*** | | | ***Productivité CH₃SH*** |
|---|---|---|---|---|---|---|
| | | | ***CH₃SH*** | ***COS*** | ***CO₂*** | ***(g.h⁻¹.L_{cat}⁻¹)*** |
| 1 | K₂MoO₄/ZrO₂ | 77 | 53 | 1 | 44 | 290 |
| 2 | K₂MoO₄/SiO₂ | 45 | 49 | 2 | 48 | 158 |
| 3 | K₂MoO₄/TiO₂ | 40 | 50 | 4 | 46 | 141 |
| 4 | K₂MoO₄/Al₂O₃ | 55 | 42 | 3 | 47 | 164 |

Les résultats présentés Tableau 1 ci-dessus montrent que le catalyseur selon l'invention (Exemple 1) donne une conversion du monoxyde de carbone et une productivité en CH₃SH nettement supérieures comparativement aux catalyseurs sur des supports de l'art antérieur (silice, titane ou alumine, exemples 2, 3 et 4).

Le catalyseur relatif à l'invention permet de synthétiser le méthylmercaptan à partir d'oxyde de carbone, d'hydrogène et d'hydrogène sulfuré avec une conversion en CO, une bonne sélectivité et une productivité accrues en MeSH, tout en combinant une meilleure conversion du COS. Ces performances améliorées sont obtenues sur un catalyseur simple, sans l'aide de promoteurs, tels que l'oxyde de tellure, l'oxyde de nickel, l'oxyde de fer, et autres, décrits dans l'art antérieur.

## Revendications

1. Procédé de préparation de méthylmercaptan, comprenant au moins les étapes suivantes :
a) réaction d'un oxyde de carbone, d'hydrogène sulfuré et d'hydrogène en présence d'un catalyseur à base de molybdène et de potassium supporté sur zircone ; ledit catalyseur ne comprenant pas de promoteur,
b) réaction d'hydrogénation de l'oxysulfure de carbone obtenu à l'étape a) en présence dudit hydrogène (H₂), pour former du méthylmercaptan (CH₃SH) et du sulfure d'hydrogène (H₂S),
c) éventuellement, recyclage dudit sulfure d'hydrogène (H2S) formé à l'étape b) vers l'étape a), et
d) récupération du méthylmercaptan.

2. Procédé selon la revendication 1, dans lequel le catalyseur utilisé dans l'étape a) comprend un composant actif à base de molybdène et de potassium au sein d'un même composant, de préférence à base de molybdène, d'oxygène et de potassium (Mo-O-K).

3. Procédé selon la revendication 1 ou la revendication 2, dans lequel le catalyseur utilisé dans l'étape a) est le tétraoxomolybdate de potassium supporté sur zircone (ZrO₂).

4. Procédé selon l'une quelconque des revendications précédentes, dans lequel le rapport pondéral du catalyseur sur zircone K₂MoO₄/ZrO₂ est compris entre 1% et 50%, de préférence de 1 à 30%, de préférence encore entre 5 et 35%.

5. Procédé selon l'une quelconque des revendications précédentes, dans lequel le support du catalyseur présente une aire spécifique supérieure à 30 m².g⁻¹, de préférence d'au moins 50 m².g⁻¹.

6. Procédé selon l'une quelconque des revendications précédentes, dans lequel l'oxyde de carbone est le monoxyde de carbone (CO).

7. Procédé selon l'une quelconque des revendications précédentes, dans lequel le sulfure d'hydrogène formé dans l'étape b) est recyclé dans l'étape a).

8. Procédé selon l'une quelconque des revendications précédentes, dans lequel la température réactionnelle dans l'étape a) est avantageusement comprise entre 100°C et 500°C, de préférence entre 200°C et 400°C, de préférence encore entre 250°C et 350°C.

9. Utilisation d'un catalyseur tel que décrit dans l'une quelconque des revendications 1 à 5, pour la production de méthylmercaptan à partir d'oxyde de carbone, de sulfure d'hydrogène et d'hydrogène.

## Patentansprüche

1. Verfahren zur Herstellung von Methylmercaptan, das mindestens die folgenden Schritte umfasst:
a) Reaktion eines Kohlenstoffoxids, von Schwefelwasserstoff und Wasserstoff in Gegenwart eines Katalysators auf Basis von Molybdän und Kalium, der auf Zirkondioxid geträgert ist; wobei der Katalysator keinen Promotor umfasst,
b) Hydrierungsreaktion des in Schritt a) erhaltenen Kohlenstoffoxysulfids in Gegenwart des Wasserstoffs (H₂), um Methylmercaptan (CH₃SH) und Schwefelwasserstoff (H₂S) zu bilden,
c) gegebenenfalls Rückführung des in Schritt b) gebildeten Schwefelwasserstoffs (H2S) zu Schritt a), und
d) Gewinnung des Methylmercaptans.

2. Verfahren nach Anspruch 1, wobei der in Schritt a) verwendete Katalysator eine Wirkkomponente auf Basis von Molybdän und Kalium innerhalb ein und derselben Komponente, vorzugsweise auf Basis von Molybdän, Sauerstoff und Kalium (Mo-O-K) umfasst.

3. Verfahren nach Anspruch 1 oder Anspruch 2, wobei es sich bei dem in Schritt a) verwendeten Katalysator um auf Zirkondioxid (ZrO₂) geträgertes Kaliumtetraoxomolybdat handelt.

4. Verfahren nach einem der vorstehenden Ansprüche, wobei das Gewichtsverhältnis des Katalysators zu Zirkondioxid K₂MoO₄/ZrO₂ im Bereich zwischen 1 % und 50 %, vorzugsweise 1 bis 30 %, weiter bevorzugt zwischen 5 und 35 % liegt.

5. Verfahren nach einem der vorstehenden Ansprüche, wobei der Träger des Katalysators eine spezifische Oberfläche von mehr als 30 m²· g⁻¹, vorzugsweise von mindestens 50 m²·g⁻¹ aufweist.

6. Verfahren nach einem der vorstehenden Ansprüche, wobei es sich bei dem Kohlenstoffoxid um Kohlenstoffmonoxid (CO) handelt.

7. Verfahren nach einem der vorstehenden Ansprüche, wobei der in Schritt b) gebildete Schwefelwasserstoff in Schritt a) wiederverwendet wird.

8. Verfahren nach einem der vorstehenden Ansprüche, wobei die Reaktionstemperatur in Schritt a) vorteilhafterweise im Bereich zwischen 100 °C und 500 °C, vorzugsweise zwischen 200°C und 400 °C, weiter bevorzugt zwischen 250°C und 350 °C liegt.

9. Verwendung eines Katalysators wie in einem der Ansprüche 1 bis 5 beschrieben zur Produktion von Methylmercaptan auf Grundlage von Kohlenstoffoxid, Schwefelwasserstoff und Wasserstoff.

## Claims

1. Process for the preparation of methyl mercaptan, comprising at least the following steps:
a) a carbon oxide, hydrogen sulphide and hydrogen undergo a reaction in the presence of a zirconia-supported molybdenum- and potassium-based catalyst; said catalyst not comprising a promoter,
b) the carbonyl sulphide obtained in step a) undergoes a hydrogenation reaction in the presence of said hydrogen (H₂), to form methyl mercaptan (CH₃SH) and hydrogen sulphide (H₂S),
c) optionally, said hydrogen sulphide (H₂S) formed in step b) is recycled to step a), and
d) the methyl mercaptan is collected.

2. Process according to claim 1, wherein the catalyst used in step a) comprises a molybdenum- and potassium-based active component within the same component, preferably a molybdenum-, oxygen- and potassium- (Mo-O-K)-based active component.

3. Process according to claim 1 or claim 2, wherein the catalyst used in step a) is zirconia- (ZrO₂)-supported potassium tetraoxomolybdate.

4. Process according to any of the preceding claims, wherein the weight ratio of the catalyst to zirconia K₂MoO₄/ZrO₂ is comprised between 1% and 50%, preferably from 1 to 30%, more preferably between 5 and 35%.

5. Process according to any of the preceding claims, wherein the catalyst support has a specific surface area greater than 30 m².g⁻¹, preferably of at least 50 m².g-¹.

6. Process according to any of the preceding claims, wherein the carbon oxide is carbon monoxide (CO).

7. Process according to any of the preceding claims, wherein the hydrogen sulphide formed in step b) is recycled in step a).

8. Process according to any of the preceding claims, wherein the reaction temperature in step a) is advantageously comprised between 100°C and 500°C, preferably between 200°C and 400°C, more preferably between 250°and to 350°C.

9. Use of a catalyst as described in any of claims 1 to 5, for producing methyl mercaptan from carbon oxide, hydrogen sulphide and hydrogen.
